# EUROPÄISCHE PATENTSCHRIFT

(11) **EP 1 789 117 B1**
(45) Veröffentlichungstag und Bekanntmachung des Hinweises auf die Patenterteilung: **12.12.2018**
(21) Anmeldenummer: 05769381.4
(22) Anmeldetag: 12.08.2005
(51) Int. Cl.: A61M 5/315, A61M 5/31

(54) **DOSIERVORRICHTUNG MIT ABLAUFSTEUERUNG**
DOSING DEVICE COMPRISING A RUN-OFF CONTROL
DISPOSITIF DE DOSAGE COMPRENANT UN ÉCOULEMENT COMMANDÉ

(30) Priorität: 25.08.2004 DE 102004041151
(43) Veröffentlichungstag der Anmeldung: 30.05.2007
(73) Patentinhaber: TecPharma Licensing AG, 3401 Burgdorf (CH)
(72) Erfinder: BURREN, Stefan, CH-3047 Bremgarten b. Bern (CH); MOSER, Ulrich, CH-3412 Heimiswil (CH); SCHRUL, Christian, CH-3400 Burgdorf (CH)
(86) Internationale Anmeldenummer: PCT/CH2005/000470
(87) Internationale Veröffentlichungsnummer: WO 2006/021110

(56) Entgegenhaltungen:
- EP-A- 0 594 349
- WO-A-03/020347
- WO-A-2004/007000
- WO-A-2004/035117
- WO-A-2005/072796
- US-A- 5 807 346
- US-A1- 2003 187 405

## Beschreibung

Die vorliegende Erfindung bezieht sich auf eine Dosiervorrichtung zur dosierten Verabreichung eines injizierbaren Produkts aus einer Injektionsvorrichtung, insbesondere auf eine Dosiervorrichtung, mit welcher eine Ablaufsteuerung bei der Abgabe einer Substanz zum Beispiel aus einer Ampulle in einer Injektionsvorrichtung realisiert werden kann, um eine Priming-Dosis und eine davon abweichende abzugebende Dosis leicht einstellen zu können.

Bei Injektionsvorrichtungen, wie zum Beispiel Injektionspens, mit welchen eine medizinische Substanz oder ein Medikament, wie zum Beispiel Insulin oder Hormone, dosiert verabreicht werden sollen, muss zunächst eine sogenannte beim Primen abzugebende Priming-Menge eingestellt werden, wobei die Injektionskanüle durch den Priming-Vorgang entlüftet und von Reststoffen befreit werden soll, indem eine bestimmte geringe Menge des zu injizierenden Produktes aus der Nadel der Injektionsvorrichtung ausgestoßen wird, ohne dabei eine Injektion vorzunehmen. Anschliessend kann eine gewöhnlich grössere Dosis der zu inji zierenden Substanz eingestellt werden.

Bei einem bekannten Injektionspen ist eine drehbare Dosierhülse vorgesehen, welche ver schiedene Anschläge aufweist und zum Beispiel als Treppenglied ausgebildet ist, wobei mit der Dosierhülse unterschiedliche Hublängen einer Zahn- oder Kolbenstange eingestellt werden können, so dass ein Benutzer meistens mindestens zwei unterschiedliche Dosis mengen einstellen muss, nämlich eine kleine Dosis der abzugebenden Substanz für das Priming und anschliessend die zu verabreichende Dosis, welche mittels der Injektionsvor- richtung injiziert werden soll.

Auch wenn immer eine konstante vorgegebene Menge oder Dosis der zu verabreichenden Substanz injiziert werden soll, ist es erforderlich, dass ein Benutzer zunächst die Priming- Menge einstellt, so dass es zu einer Fehlbedienung seitens des Benutzers kommen kann und beispielsweise die auszuschüttende Dosis für das Priming verwendet wird oder sogar die Priming-Dosis injiziert wird.

Aus der US5807346 und US2003/0187405 sind sogenannte Zahnstangenpens bekannt, bei der eine Vorschubhülse für eine Aufziehbewegung in eine Aufziehrichtung relativ zu einer Aussenhülse und einer von Rastelementen der Aussenhülse gehaltenen Zahnstange bewegbar ist.

Es ist eine Aufgabe der vorliegenden Erfindung eine Dosiervorrichtung zur dosierten Ver abreichung eines injizierbaren Produkts aus einer Injektionsvorrichtung vorzuschlagen, welche eine benutzerfreundliche Bedienung einer Injektionsvorrichtung insbesondere beim Primen ermöglicht.

Diese Aufgabe wird durch eine Dosiervorrichtung nach Anspruch 1 gelöst. Vorteilhafte Ausführungsformen ergeben sich aus den Unteransprüchen.

Die erfindungsgemäße Dosiervorrichtung zur dosierten Verabreichung eines injizierbaren Produktes aus einer Injektionsvorrichtung weist eine Außenhülse und eine Vorschubhülse auf, welche relativ zur Außenhülse bewegbar ist. Erfindungsgemäß ist an der Vorschubhülse mindestens ein verschiebbares und flexibles Element mit einem ersten Anschlag vorgesehen, welcher an einem Gegenanschlag der Außenhülse anliegen und eine Relativbewegung zwischen der Außenhülse und der Vorschubhülse begrenzen kann. Ein solches erfindungsgemäß vorgesehenes verschiebbares und flexibles Element mit einem ersten Anschlag ermöglicht es, dass, wenn ein Profil in einer Kolben- oder Zahnstange oder einem anderen Vorschubelement vorgesehen ist, welches durch eine Bewegung das Ausschütten einer Substanz aus einer Ampulle zum Beispiel durch Verschieben eines Kolbens bewirken kann, das flexible Element oder ein daran vorgesehenes Eingriffsstück in eine Vertiefung zum Beispiel einer Zahnstange eingreifen kann, um den ersten Anschlag in eine Position zu bringen, in welcher die Vorschubhülse frei relativ zur Außenhülse bewegt werden kann. Wird das verschiebbare und flexible Element oder das daran angebrachte Eingriffsstück durch eine Rampe oder ein anderes Profil der Zahnstange oder eines anderen verschiebbaren Elements durch eine Verschiebung der Zahnstange relativ zur Vorschubhülse verschoben, also zum Beispiel in radialer Richtung durch eine Rampe einer Zahnstange nach außen gedrückt, so kann der erfindungsgemäß auf dem Vorschubelement vorgesehene erste Anschlag in eine Position gebracht werden, welche die Relativbewegung zwischen Außenhülse und Vorschubhülse dadurch begrenzen kann, dass ein an der Außenhülse vorgesehener Gegenanschlag an den Anschlag der Vorschubhülse bewegt wird und bei Anlage die Bewegung stoppt. Insbesondere kann der erste Anschlag so an der Vorschubhülse vorgesehen sein, dass die Vorschubhülse nur soweit bewegt werden kann, bis der Gegenanschlag der Außenhülse an dem ersten Anschlag der Vorschubhülse anliegt und somit eine weitere Bewegung der Vorschubhülse begrenzen kann. Es ist auch möglich, dass der erste Anschlag nicht erst durch eine Rampe einer Zahnstange in eine Anschlagposition gedrückt wird, sondern so angeordnet ist, dass nach der Bewegung der Vorschubhülse relativ zur Außenhülse eine Blockade einer weiteren Bewegung realisiert werden kann. Ein Anschlag kann im Sinne der Erfindung jedes Element sein, welches eine Relativbewegung zweier Objekte oder Hülsen einschränken oder unterbrechen kann.

Wird die Vorschubhülse, welche zum Beispiel mit einem Knopf zur einfacheren Bedienung durch den Benutzer verbunden sein kann, während einer Aufziehbewegung der Injektionsvorrichtung, also zum Beispiel durch ein Herausziehen des Knopfes, nach hinten gezogen, also zu einem der Abgabeseite der Injektionsvorrichtung entgegengesetzten Ende der Injektionsvorrichtung, so kann der erste Anschlag der Vorschubhülse bewirken, dass die Injektionsvorrichtung nur soweit aufgezogen werden kann bis dieser an einem Gegenanschlag der Außenhülse anliegt, das heißt der Abstand zwischen dem Anschlag auf der Vorschubhülse und dem Gegenanschlag der Außenhülse bestimmt die maximal mögliche Strecke einer Aufzugbewegung der Injektionsvorrichtung und damit die maximale Menge einer Dosis einer abzugebenden Substanz. Bevorzugt ist ein Profil oder eine Vertiefung in der Zahnstange nur über eine kleinere Teilstrecke vorgesehen, so dass das flexible Element der Vorschubhülse nach einer relativ kleinen Aufzugbewegung schon nach außen gedrückt wird, wodurch für einen ersten Abgabe- oder Ausschüttvorgang festgelegt werden kann, dass nur eine kleine Menge, zum Priming, abgegeben wird. Somit muss ein Benutzer die Priming-Dosis nicht mehr separat einstellen, da die automatische Begrenzung durch die erfindungsgemäße Dosiervorrichtung sicherstellen kann, dass ein Benutzer keine größere Dosis als die durch den Abstand der Anschläge definierte Priming-Dosis für einen ersten Abgabevorgang einstellen kann. Für nachfolgende Abgabevorgänge zum Injizieren einer Dosis kann zum Beispiel nach einer Rampe in einer Zahnstange eine längere Vertiefung in der Zahnstange vorgesehen sein, so dass größere Mengen einer abzugebenden Substanz dosiert bzw. eingestellt werden können. Somit wird durch die erfindungsgemäße Dosiervorrichtung die Wahl einer zuerst abzugebenden Dosismenge automatisch gesteuert, so dass ein Benutzer einen Priming-Vorgang mit einer kleinen Dosis und eine nachfolgende Einstellung einer Dosis zur Injektion durchführen kann, ohne die Priming-Dosis selbst einstellen zu müssen. Ein Benutzer hat somit bezüglich der Priming-Dosis keine Einstellmöglichkeit mehr, wodurch Fehlmanipulationen oder Fehleinstellungen einer Injektionsvorrichtung weitgehend verhindert werden können und ein Benutzer den Bedienungsablauf bei der Verwendung der Injektionsvorrichtung leicht beherrschen kann.

Eine erfindungsgemäße Außenhülse kann um die Vorschubhülse herum oder auch innerhalb der Vorschubhülse angeordnet sein und ist vorteilhaft mit der Außenseite einer Injektionsvorrichtung verbunden oder bildet zumindest einen Teil davon, während die Vorschubhülse vorteilhaft mit einem Einstellelement, wie zum Beispiel einem Knopf, verbunden ist.

Bevorzugt können zwei oder mehr flexible Elemente an der Vorschubhülse vorgesehen sein, wobei ein flexibles Element zum Beispiel als elastischer Arm ausgebildet sein kann, welcher zum Beispiel in einer Ausnehmung oder Öffnung der Vorschubhülse angeordnet ist und vorteilhaft im Ausgangszustand in Richtung einer Zahnstange oder Kolbenstange drückt, welche beispielsweise innerhalb der Vorschubhülse geführt wird. Ein an der Vorschubhülse zum Beispiel am Arm vorgesehenes Eingriffsstück weist dabei vorteilhaft in Richtung der Zahn- oder Kolbenstange, das heißt zum Beispiel bei einer etwa zylinderförmigen Vorschubhülse nach innen.

Der auf dem flexiblen Element oder der Vorschubhülse vorgesehene erste Anschlag kann bei einer etwa zylinderförmigen Vorschubhülse sowohl nach innen, als auch nach außen weisen und ist zum Beispiel als Nase oder Nocken ausgebildet, so dass der Anschlag in Anlage mit einem entsprechenden Gegenanschlag der Außenhülse kommen kann, um eine Relativbewegung zum Beispiel in axialer Richtung der zum Beispiel in der Außenhülse verschiebbar gelagerten Vorschubhülse nur bis zum Aufeinandertreffen der Anschläge zu ermöglichen und hierdurch eine Priming-Dosis automatisch festzulegen.

Auf der Vorschubhülse ist noch mindestens ein zweiter Anschlag zum Beispiel in Form einer vorstehenden Nase oder eines Nockens vorgesehen, wobei der zweite Anschlag eine Relativbewegung zwischen Außenhülse und Vorschubhülse begrenzen kann, wenn keine Begrenzung durch den ersten Anschlag der Vorschubhülse stattfindet. Wenn das flexible Element der Vorschubhülse, auf welchem der erste Anschlag angeordnet ist, durch die Vorspannung des flexiblen Elements in eine Vertiefung einer Zahnstange eingreift und somit das Einstellen einer größeren Dosis als der Priming-Dosis ermöglicht, kann durch den Abstand zwischen dem zweiten Anschlag der Vorschubhülse und einem Gegenanschlag der Außenhülse festgelegt werden, wie groß eine maximal einzustellende Dosis zum Beispiel für einen Injektionsvorgang sein kann. Der erste und zweite Anschlag sind in axialer Richtung der Vorschubhülse versetzt zueinander angeordnet und können zum Beispiel mit dem gleichen oder auch mit verschiedenen Anschlägen der Außenhülse zusammenwirken.

Die Erfindung bezieht sich weiterhin auf eine wie oben beschriebene Dosiervorrichtung mit einer Zahnstange, welche durch die Außenhülse und/oder Vorschubhülse und bevorzugt innerhalb der Vorschubhülse geführt wird und welche mindestens eine und bevorzugt zwei oder mehrere Vertiefungen aufweist, welche ein Abgabeprofil definieren können. Sind mindestens zwei, zum Beispiel in axialer Richtung der Zahn- oder Kolbenstange hintereinanderliegende Vertiefungen vorgesehen, so sind diese einzelnen Vertiefungen bevorzugt durch eine Rampe oder eine Erhöhung voneinander getrennt, so dass ein in die Vertiefungen eingreifendes Eingriffsstück oder flexibles Element der Vorschubhülse durch eine solche Rampe oder Erhöhung von der Mittelachse der Zahnstange oder Kolbenstange weg nach außen verschoben werden kann, so dass der auf der Vorschubhülse und dem flexiblen Element vorgesehene erste Anschlag radial nach außen weg von der Zahnstange gedrückt wird, um in Verbindung mit einem Gegenanschlag der Außenhülse eine Begrenzung der Relativbewegung zwischen Außen- und Vorschubhülse und damit eine. Begrenzung einer Aufziehbewegung der Injektionsvorrichtung zur Einstellung oder Festlegung einer Priming-Dosis zu realisieren. Prinzipiell kann auch nur eine einzige Vertiefung in einer Zahnstange vorgesehen sein, in welche das flexible Element der Vorschubhülse nach dem Priming eingreifen kann, so dass der erste Anschlag die Einstellung einer weiteren Dosis nicht beeinflussen kann.

Vorzugsweise sind an der Außenhülse und/oder der Vorschubhülse oder einem mit der Vorschubhülse verbundenen Führungsstück Rastelemente vorgesehen, welche vorteilhaft an elastischen oder federnden Elementen oder Armen angebracht sein können, wobei die Rastelemente vorzugsweise so ausgebildet sind, dass eine Relativbewegung einer Zahnstange, in welche diese Rastelemente eingreifen, in einer Richtung ermöglicht und in einer anderen Richtung blockiert wird.

Bevorzugt sind die Rastelemente so ausgebildet, dass sie in entsprechende Vertiefungen oder eine Zahnung einer Zahnstange so eingreifen, dass die Zahnstange in Richtung einer Abgabeöffnung der Injektionsvorrichtung relativ zu der Außenhülse oder dem Führungsstück bzw. relativ zu der Vorschubhülse bewegt werden kann.

Gemäß einem weiteren Aspekt bezieht sich die Erfindung auf eine Injektionsvorrichtung mit einer wie oben beschriebenen Dosiervorrichtung.

Weiterhin beschrieben wird ein Verfahren zum Primen oder zur Vorbereitung der dosierten Verabreichung eines injizierbaren Produktes aus einer wie oben beschriebenen Injektionsvorrichtung, wobei eine erste Aufziehbewegung der Injektionsvorrichtung nach einer ersten vorgegebenen Distanz durch ein Anliegen eines ersten Anschlags einer Vorschubhülse an einem Gegenanschlag einer Außenhülse begrenzt wird und eine erste Dosis, eine Priming-Dosis, durch Verschieben einer Zahnstange oder Kolbenstange zum Primen abgegeben wird und wobei die Begrenzung der Relativbewegung zwischen Außenhülse und Vorschubhülse durch den ersten Anschlag der Vorschubhülse während oder nach dem Abgeben der Priming-Dosis freigegeben wird.

Die Erfindung wird nachfolgend anhand eines bevorzugten Ausführungsbeispiels beschrieben werden. Es zeigen:
- Figuren 1A bis 1E: eine erfindungsgemäße Dosiervorrichtung mit einer Zahnstange in verschiedenen Betriebszuständen; und
- Figuren 2 und 3: Querschnittsansichten des in eine Zahnstange eingreifenden flexiblen Elements der Vorschubhülse.

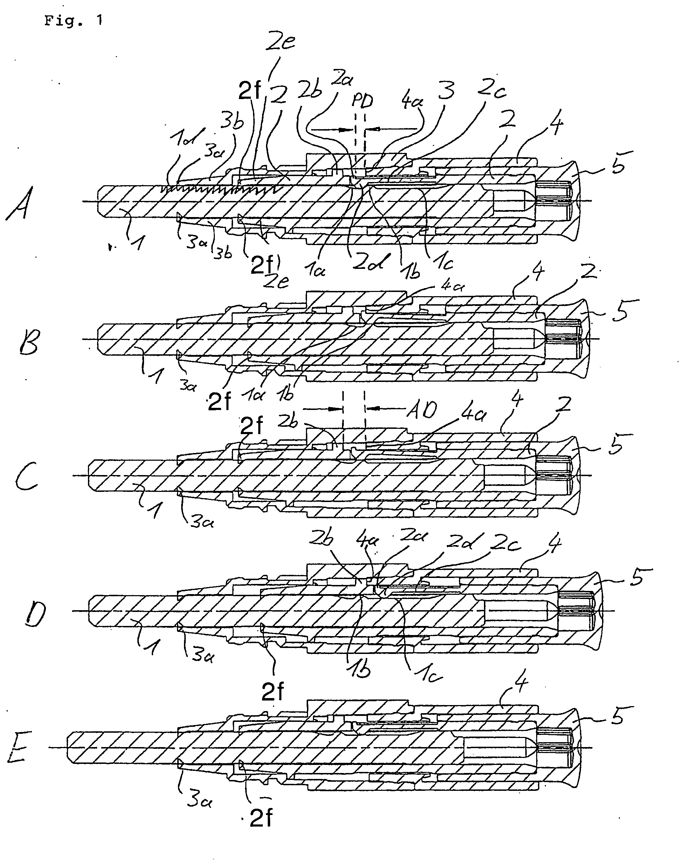

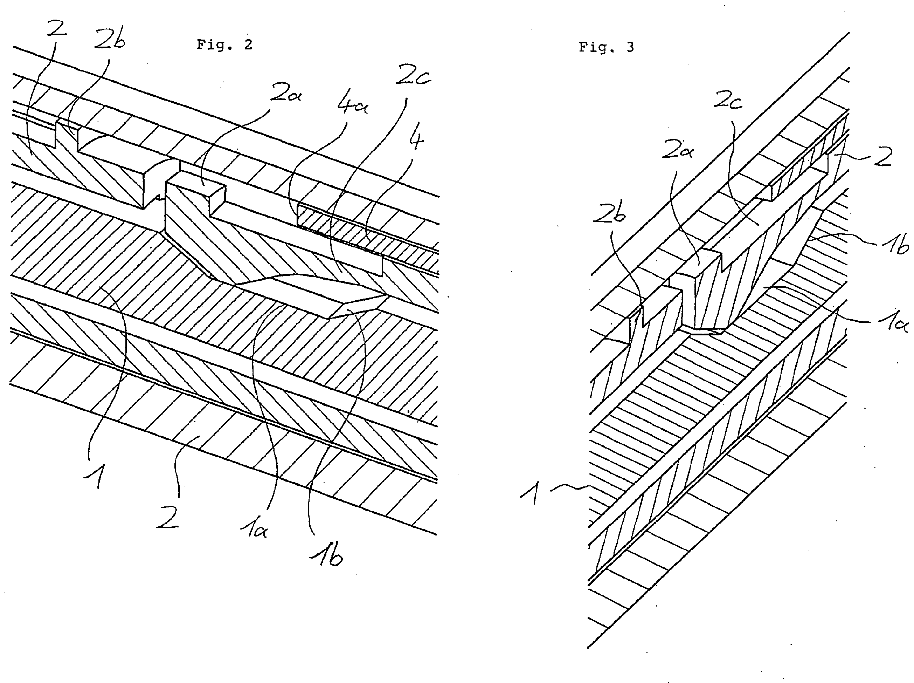

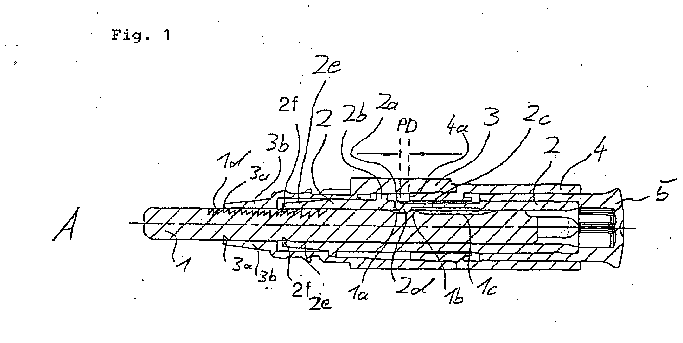

Figur 1A zeigt eine erfindungsgemäße Dosiervorrichtung in Grundstellung, welche zum Beispiel das hintere Ende einer Injektionsvorrichtung bilden kann, mit einer innerhalb einer Vorschubhülse 2 angeordneten Zahnstange 1 mit einer nur auf der Oberseite schematisch dargestellten Zahnung 1d, welche an der in Figur 1A links gezeigten Vorderseite auf einen Stopfen einer Ampulle (nicht gezeigt) drücken kann, um durch eine Bewegung der Zahnstange 1 in Figur 1A nach links einen Ausschüttvorgang einer Injektionsvorrichtung zu bewirken. Um die Zahnstange 1 herum ist die Vorschubhülse 2 angeordnet, welche erfindungsgemäß ein flexibles Element 2c, welches ein elastisch gelagerter Arm ist, aufweist, das wie auch in den Figuren 2 und 3 gezeigt, in einer Öffnung der Vorschubhülse 2 vorgesehen ist und sich radial nach innen oder außen bewegen kann und in der in Figur 1A gezeigten Grundstellung in eine erste Vertiefung la der Zahnstange 1 eingreift, so dass in der gezeigten Grundstellung das flexible Element 2c nicht vorgespannt ist, was vorteilhaft ist, da bei einer zum Beispiel aus Kunststoff gefertigten Vorschubhülse 2 die Spannung eines vorgespannten flexiblen Elements 2c mit der Zeit nachlassen würde. Die Vorschubhülse 2 weist an der in Figur 1A links gezeigten Vorderseite elastische Elemente, wie zum Beispiel Federarme 2e auf, welche an ihrem Vorderende nach innen weisende Rastelemente 2f tragen, die in Vertiefungen oder die Zahnung 1d der Zahnstange 1 eingreifen können, so dass die Zahnstange 1 in Figur 1A nur in Richtung der Abgabeöffnung einer Injektionsvorrichtung, dass heißt in Figur 1A nach links, relativ zur Vorschubhülse 2 verschoben werden kann, wobei eine Bewegung in entgegengesetzter Richtung durch die in auf der Außenseite der Zahnstange 1 liegende Vertiefungen bzw. Verzahnungen 1d eingreifenden Rastelemente 2f blockiert wird. Die Vorschubhülse 2 ist fest mit einem als Einstellelement dienenden Knopf 5 verbunden.

Um die Vorschubhülse 2 herum ist eine Außenhülse 4 vorgesehen, welche einen feststehenden Anschlag 4a aufweist, welcher nicht durch eine Betätigung des Knopfes 5 verschoben werden kann. Die Außenhülse 4 ist mit einem Führungsstück 3 verbunden, welches ebenfalls wie der vordere Teil der Vorschubhülse 2 elastische Arme 3b mit Rastelementen 3a aufweist, die wie oben beschrieben in die Zahnstange 1 eingreifen und nur eine Relativbewegung in eine Richtung ermöglichen.

Die Zahnstange 1 weist in Längsrichtung längliche Vertiefungen 1a und 1c auf, welche durch eine Rampe 1b voneinander getrennt sind. Das flexible Element 2c der Vorschubhülse greift in der gezeigten Grundstellung in die erste Vertiefung la der Zahnstange 1 ein, so dass ein auf der Außenseite des flexiblen Elements 2c vorgesehener erster Anschlag 2a radial nicht über den Umfang der Vorschubhülse 2 hinausragt.

Figur 1B zeigt den Zustand der in Figur 1A gezeigten Dosiervorrichtung, nachdem ein Benutzer die Dosiervorrichtung durch einen Zug an dem Knopf 5, in Figur 1B nach rechts, aufgezogen hat. Die mit dem Knopf 5 verbundene Vorschubhülse 2 wird hierdurch in Aufziehrichtung relativ zur Außenhülse 4 und der von den Rastelementen 3a gehaltenen Zahnstange 1 bewegt, wobei das an dem flexiblen Element 2c vorgesehene Eingriffsstück 2d auf der Rampe 1b der Zahnstange 1 entlang wandert und somit radial nach außen gedrückt wird, so dass die auf der Außenseite des flexiblen Elements 2c vorgesehene als Anschlag dienende Nase 2a radial nach außen gedrückt wird und nach der Aufziehdistanz PD an dem Anschlag 4a der Außenhülse 4 anliegt, wodurch die Aufziehbewegung durch Ziehen an dem Knopf 5 relativ zur Außenhülse 4 begrenzt wird. Der in Figur 1A gezeigte Abstand PD zwischen dem ersten Anschlag 2a der Vorschubhülse 2 und dem feststehenden Anschlag 4a der Außenhülse 4 legt somit die maximale Größe der Aufziehbewegung und damit die maximale Menge der Priming-Dosis fest. Während der Aufziehbewegung zieht ein Benutzer den Knopf 5 aus der Außenhülse 4 heraus, wobei die Zahnstange 1 durch die an dem mit der Außenhülse 4 fest verbundenen Führungsstück 3 vorgesehenen Rastelemente 3a festgehalten wird, so dass sich die Zahnstange 1 relativ zum Führungsstück 3 bzw. zur Außenhülse 4 während des Aufziehvorgangs nicht bewegt. Die Rastelemente 2f an den elastischen Armen 2e der Vorschubhülse 2 wandern dabei entlang des Zahnstangenprofils 1d in axialer Richtung der Zahnstange 1 nach hinten. Durch einen Druck auf den Knopf 5 wird dieser zum Ausschütten oder Primen wieder in die Außenhülse 4 hinein gedrückt, wie in Figur 1C gezeigt, wobei die auf der Vorderseite der Vorschubhülse 2 vorgesehenen Rastelemente 2c in die Zahnstange 1 eingreifen und den auf die mit dem Knopf 5 verbundene Vorschubhülse 2 ausgeübten Druck auf die Zahnstange 1 übertragen, so dass diese in Figur 1C nach links verschoben wird, um auf einen nicht gezeigten Stopfen einer Ampulle zu drücken, wodurch die Abgabe der Priming-Dosis bewirkt wird. Dabei wird die Vorschubhülse 2 relativ zur Zahnstange nicht bewegt. Die Rastelemente 3a wandern entlang des Zahnprofils 1d der Zahnstange 1.

Nach dem Primen bzw. dem Ausschütten der durch den Abstand PD festgelegten Priming-Dosis kann die Dosiervorrichtung zur Einstellung einer für eine Injektion bestimmten Dosis aufgezogen werden, wie in Figur 1D gezeigt. Dabei wird die Vorschubhülse 2 erneut durch ein Herausziehen des Knopfes 5 relativ zu der von den Rastelementen 3a des Führungsstückes 3 gehaltenen Zahnstange 1 nach hinten bewegt. Das Eingriffsstück 2d wird dabei von der Rampe 1b der Zahnstange 1 weg in Richtung der zweiten Vertiefung 1c der Zahnstange 1 geschoben und durch die elastische Kraft des flexiblen Elements 2c in radialer Richtung nach innen in die zweite Vertiefung 1c hineingedrückt, so dass die den ersten Anschlag bildende Nase 2a von dem feststehenden Anschlag 4a der Außenhülse 4 weg bewegt wird und somit eine größere Dosis eingestellt werden kann als diejenige, welche durch den Abstand PD definiert wurde. Beim Aufziehen kann die Vorschubhülse 2 so weit verschoben werden bis der als zweiter Anschlag auf der Außenseite der Vorschubhülse 2 vorgesehene Nocken 2b an dem feststehenden Anschlag 4a der Außenhülse 4 anliegt und somit die Aufziehbewegung begrenzt. Der in Figur 1C eingezeichnete Abstand AD zwischen dem zweiten Anschlag 2b der Vorschubhülse 2 und dem Anschlag 4a der Außenhülse 4 definiert somit die maximale Größe einer Aufziehbewegung und hierdurch eine Ausschütt-Dosis für eine Injektion. Die Aufziehbewegung zur Einstellung der Abgabe-Dosis wird somit nicht mehr durch den ersten Anschlag 2a begrenzt. Wie oben für das Aufziehen zur Durchführung des Primings beschrieben, halten die Rastelemente 3a des Führungsstückes 3 die Zahnstange 1, während die Rastelemente 2f der Vorschubhülse 2 entlang der Zahnstange 1 wandern.

Anschließend kann eine eingestellte Dosis durch Druck auf den Knopf 5 und Verschieben der Vorschubhülse 2 ausgeschüttet werden, wie in Figur 1E gezeigt. Dabei wird die Zahnstange 1 durch die Rastelemente 2f der Vorschubhülse 2 zusammen mit der Vorschubhülse 2 in Richtung einer Abgabeöffnung der Injektionsvorrichtung verschoben, wobei die Rastelemente 3a des Führungsstückes 3 entlang der Zahnstange 1 wandern. Somit erfolgt eine Ablaufsteuerung durch das Aus- und Einfahren des ersten Anschlages 2a auf dem flexiblen Element 2c der Vorschubhülse 2 mittels des durch die Vertiefungen 1a und 1c bzw. der Rampe 1b definierten Profils in der Zahnstange 1. Durch die Länge und/oder Anordnung verschiedener Vertiefungen oder Profile in der Zahnstange 1 kann die Abfolge von verschiedenen Dosierungen eingestellt werden, wobei beispielsweise weitere Anschläge zusätzlich zu dem einen im Ausführungsbeispiel gezeigten fixen Anschlag 4a der Außenhülse 4 zum Beispiel in entsprechenden Vertiefungen und/oder Ausnehmungen der Außenhülse 4 vorgesehen sein können, oder weitere Rampen und Vertiefungen im Profil der Zahnstange 1 vorgesehen sein können.

## Patentansprüche

1. Dosiervorrichtung zur dosierten Verabreichung eines injizierbaren Produktes aus einer Injektionsvorrichtung mit einer Aussenhuelse (4), einer relativ zur Aussenhuelse (4) bewegbaren Vorschubhuelse (2), wobei die Vorschubhuelse (2) mindestens ein verschiebbares und flexibles Element (2c) mit einem ersten Anschlag (2a) aufweist, der an einem Gegenanschlag (4a) der Aussenhuelse (4) anliegen und eine Relativbewegung zwischen der Aussenhuelse (4) und der Vorschubhuelse (2) begrenzen kann,
wobei das flexible Element (2c) ein in einer Ausnehmung der Vorschubhü|se(2) elastisch gelagerter Arm ist, **dadurch gekennzeichnet, dass** auf der Vorschubhuelse (2) ein zweiter Anschlag (2b) vorgesehen ist,
wobei der erste (2a) und zweite Anschlag (2b) in axialer Richtung der Vorschubhuelse (2) versetzt zueinander angeordnet sind,
und mit dem ersten Anschlag (2a) eine Priming-Dosis automatisch festgelegt wird, und dem zweiten Anschlag (2b) eine maximal einzustellende Dosis festlegt wird.

2. Dosiervorrichtung nach dem vorhergehenden Anspruch mit einem Einstellelement oder Knopf (5), welcher fest mit der Vorschubhuelse (2) verbunden ist.

3. Dosiervorrichtung nach einem der vorhergehenden Ansprueche, wobei der erste Anschlag (2a) ein von dem flexiblen Element (2c) vorstehender Nocken und/oder eine Nase ist

4. Dosiervorrichtung nach einem der vorhergehenden Ansprueche, wobei die Vorschubhuelse (2) in der Aussenhuelse (4) verschiebbar gelagert ist.

5. Dosiervorrichtung nach einem der vorhergehenden Ansprueche mit einer Zahnstange (1), welche mindestens eine Vertiefung (Ia, Ic) aufweist.

6. Dosiervorrichtung nach dem vorhergehenden Anspruch, wobei die Zahnstange (1) mindestens zwei Vertiefungen (Ia, Ic) aufweist, welche durch eine Rampe (Ib) voneinander in axialer Richtung der Zahnstange getrennt sind.

7. Dosiervorrichtung nach einem der vorhergehenden Ansprueche, wobei die Vorschubhuelse (2), die Aussenhuelse (4) und/oder ein mit der Aussenhuelse (4) verbundenes Fuehrungsstueck (3) Rastelemente (2c, 3a) aufweisen, welche bevorzugt an Federarmen (2e, 3b) der jeweiligen Huelsen oder Stuecke (2, 3, 4) angebracht sind und in eine Zahnung der Zahnstange (1) eingreifen koennen.

8. Injektionsvorrichtung mit einer Dosiervorrichtung nach einem der vorhergehenden Ansprueche.

## Claims

1. A dosing device for the dosed administration of an injectable product from an injection device, comprising an outer sleeve (4) and an advance sleeve (2) displaceable relative to the outer sleeve (4), wherein the advance sleeve (2) has at least one displaceable and flexible element (2c) having a first abutment (2a) which bears against a counterpart abutment (4a) of the outer sleeve (4) and can delimit a relative movement between the outer sleeve (4) and the advance sleeve (2), wherein the flexible element (2c) is an arm which is elastically mounted in an opening in the advance sleeve (2), **characterised in that** a second abutment (2b) is provided on the advance sleeve (2), wherein the first (2a) and second (2b) abutments are arranged in mutually displaced relationship in the axial direction of the advance sleeve (2) and a priming dose is automatically established with the first abutment (2a) and a maximum dose to be set is established with the second abutment (2b).

2. A dosing device according to the preceding claim comprising a setting element or knob (5) fixedly connected to the advance sleeve (2).

3. A dosing device according to one of the preceding claims wherein the first abutment (2a) is a projection and/or a nose projecting from the flexible element (2c).

4. A dosing device according to one of the preceding claims wherein the advance sleeve (2) is displaceably mounted in the outer sleeve (4).

5. A dosing device according to one of the preceding claims comprising a toothed bar (1) which has at least one recess (1a, 1c).

6. A dosing device according to the preceding claim wherein the toothed bar (1) has at least two recesses (1a, 1c) which are separated from each other in the axial direction of the toothed bar by a ramp (1b).

7. A dosing device according to one of the preceding claims wherein the advance sleeve (2), the outer sleeve (4) and/or a guide portion (3) connected to the outer sleeve (4) have latch elements (2c, 3a) which are preferably mounted to spring arms (2e, 3b) of the respective sleeves or portions (2, 3, 4)and can engage into a tooth arrangement of the toothed bar (1).

8. An injection device comprising a dosing device according to one of the preceding claims.

## Revendications

1. Dispositif de dosage pour l'administration dosée d'un produit injectable provenant d'un dispositif d'injection avec une douille extérieure (4), une douille d'avancée (2) mobile par rapport à la douille extérieure (4), dans lequel la douille d'avancée (2) présente au moins un élément (2c) pouvant être déplacé et flexible avec une première butée (2a) qui peut être appliquée sur une contrebutée (4a) de la douille extérieure (4) et peut délimiter un mouvement relatif entre la douille extérieure (4) et la douille d'avancée (2),
dans lequel l'élément flexible (2c) est un bras monté de manière élastique dans une cavité de la douille d'avancée (2),
**caractérisé en ce que**
sur la douille d'avancée (2), une deuxième butée (2b) est prévue,
dans lequel la première (2a) et la deuxième butée (2b) sont disposées dans la direction axiale de la douille d'avancée (2) de manière décalée l'une par rapport à l'autre,
et, avec la première butée (2a), une dose d'amorçage est déterminée automatiquement et, avec la deuxième butée (2b), une dose à ajuster de manière maximale est déterminée.

2. Dispositif de dosage selon la revendication précédente avec un élément d'ajustement ou un bouton (5) qui est relié fixement à la douille d'avancée (2).

3. Dispositif de dosage selon l'une quelconque des revendications précédentes, dans lequel la première butée (2a) est une came dépassant de l'élément flexible (2c) et/ou un bec.

4. Dispositif de dosage selon l'une quelconque des revendications précédentes, dans lequel la douille d'avancée (2) est montée de manière à pouvoir être déplacée dans la douille extérieure (4).

5. Dispositif de dosage selon l'une quelconque des revendications précédentes, avec une crémaillère (1) qui présente au moins un creux (1a, 1c).

6. Dispositif de dosage selon la revendication précédente, dans lequel la crémaillère (1) présente au moins deux creux (1a, 1c) qui sont séparés l'un de l'autre par une rampe (1b) dans la direction axiale de la crémaillère.

7. Dispositif de dosage selon l'une quelconque des revendications précédentes, dans lequel la douille d'avancée (2), la douille extérieure (4) et/ou une pièce de guidage (3) reliée à la douille extérieure (4) présentent des éléments d'encliquetage (2c, 3a) qui sont attachés de préférence à des bras à ressort (2e, 3b) des douilles ou pièces (2, 3, 4) respectives et peuvent venir en prise avec une denture de la crémaillère (1).

8. Dispositif d'injection avec un dispositif de dosage selon l'une quelconque des revendications précédentes.
